(19) Europäisches Patentamt
European Patent Office
Office européen des brevets

(11) Veröffentlichungsnummer : **0 035 624**
**B1**

# (12) EUROPÄISCHE PATENTSCHRIFT

(45) Veröffentlichungstag der Patentschrift : **04.07.84**

(51) Int. Cl.³ : **C 07 C149/32**, C 07 D295/10// C07D239/48

(21) Anmeldenummer : **81100160.1**

(22) Anmeldetag : **03.11.78**

(60) Veröffentlichungsnummer der früheren Anmeldung nach Art. 76 EPÜ : **0003212**

## (54) Aldehyde.

(30) Priorität : **10.11.77 LU 78487**
**19.09.78 CH 9776/78**

(43) Veröffentlichungstag der Anmeldung : **16.09.81 Patentblatt 81/37**

(45) Bekanntmachung des Hinweises auf die Patenterteilung : **04.07.84 Patentblatt 84/27**

(84) Benannte Vertragsstaaten : **BE CH DE FR LU NL SE**

(56) Entgegenhaltungen :
**DE-A- 2 202 715**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Kompis, Ivan, Dr.**
**Lettenhofstrasse 6**
**CH-4104 Oberwil (CH)**
Erfinder : **Wick, Alexander Eduard, Dr.**
**Rue du Buisson Richard 10**
**F-78600 Le Mesnil-le-Roy (FR)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 035 624 B1

**Beschreibung**

Die vorliegende Erfindung betrifft neue Aldehyde der allgemeinen Formel

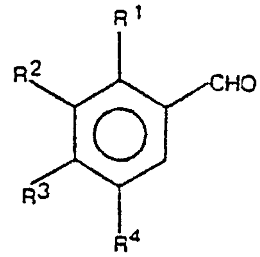

(VIII)

worin

$R^1$ Wasserstoff ; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff ; $R^2$ $C_{1-6}$-Alkoxy und $R^3$ und $R^4$ $C_{1-6}$-Alkylthio oder
$R^1$ Wasserstoff ; $R^2$ und $R^4$ $C_{1-6}$-Alkoxy und $R^3$ $C_{1-6}$-Alkylthio, Carboxy-$C_{1-6}$-alkylthio oder Cyano-$C_{1-6}$-alkylthio darstellen.

Die allgemeine Formel VIII umfasst die Verbindungsgruppen der Formeln VIII-3 bis VIII-5 :

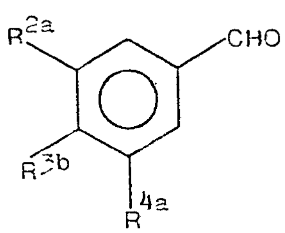

(VIII-3)

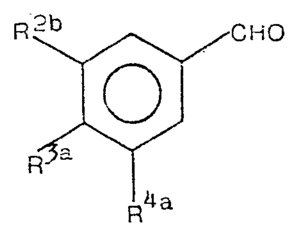

(VIII-4)

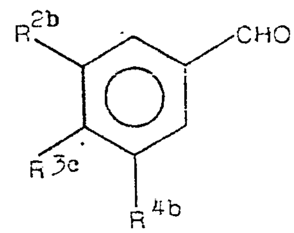

(VIII-5)

wobei

$R^{2a}$, $R^{3a}$ und $R^{4a}$ $C_{1-6}$-Alkylthio,
$R^{2b}$ und $R^{4b}$ $C_{1-6}$-Alkoxy,
$R^{3b}$ $C_{1-6}$-Alkoxy, $C_{1-6}$-Alkylthio oder Pyrrolo und
$R^{3c}$ $C_{1-6}$-Alkylthio, Carboxy-$C_{1-6}$-alkylthio oder Cyano-$C_{1-6}$-alkylthio bedeuten.

Besonders bevorzugte Aldehyde der Formel VIII sind 3,5-Dimethoxy-4-methylthiobenzaldehyd und 4-Ethylthio-3,5-dimethoxybenzaldehyd.

Die Herstellung der Aldehyde der Formel VIII aus bekannten Verbindungen kann in an sich bekannter Weise, z. B. wie in den folgenden Reaktionsübersichten I-III schematisch dargestellt, und wie in den Beispielen 1-5 im Detail beschrieben, erfolgen.

Die Thermolyse einer Verbindung XX zu einem Aldehyd VIII-5 (Reaktionsschema II) wurde in der Literatur bisher noch nicht beschrieben und ist noch neu. Diese Reaktion wird zweckmässigerweise in Dimethylformamid bei einer Temperatur von ca. 50 °C bis zur Rückflusstemperatur des Reaktionsgemisches durchgeführt. Durch Spuren von Alkalimetallxanthogenat, beispielsweise Kaliumxanthogenat, wird die Reaktion katalysiert.

Die Aldehyde der Formel VIII können via Verbindungen der Formel

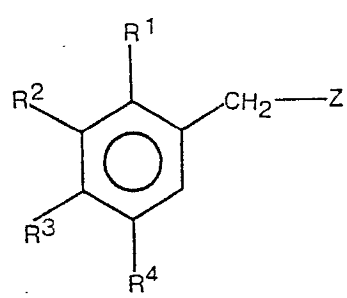

(II)

in pharmakologisch aktive 2,4-Diamino-5-benzylpyrimidine der Formel

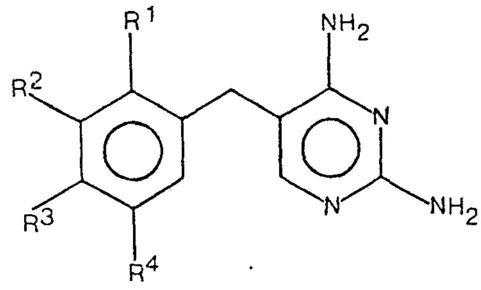

(I)

worin $R^1$, $R^2$, $R^3$ und $R^4$ die oben angegebenen Bedeutungen haben, Z eine Gruppe —C(CN) = CH—Y oder —CH(CN)CH$(OR^5)_2$, Y eine Abgangsgruppe und $R^5$ $C_{1-6}$-Alkyl, 2-$C_{1-6}$-Alkoxyäthyl, 2-Phenoxyäthyl oder 2-Benzyloxyäthyl oder beide Reste $R^5$ gemeinsam $C_{1-6}$-Alkylen darstellen, übergeführt werden.

Die Verbindungen der Formel I selbst sind Gegenstand der europäischen Patentanmeldung Nr. 78.101300.8.

Die Herstellung der Verbindungen II aus den Aldehyden der Formel VIII kann, wie im Reaktionsschema IV dargestellt, erfolgen, wobei die einzelnen Reaktionsschritte in Analogie zu bekannten Umsetzungen durchgeführt werden können.

So kann z. B. ein Aldehyd der Formel VIII mit einem β-Alkoxypropionitril der Formel $NC—CH_2—CH_2—OR^5$ zu einer Verbindung der Formel VI und in Gegenwart eines Alkalimetallalkoxides wie Natriummethoxid oder Kalium-tert.-butylat und eines Alkanols wie Methanol, Aethanol oder Propanol zu einer Verbindung der Formel II-a umgesetzt werden. Die α,β-ungesättigte Verbindung VI steht im Gleichgewicht mit der entsprechenden β,γ-ungesättigten Verbindung II-e, die mit Guanidin zu einer Verbindung I umgesetzt werden kann.

Setzt man einen Aldehyd der Formel VIII zunächst mit Malodinitril unter den Bedingungen einer Knoevenagel-Kondensation (ggf. basischer Katalysator) um, so erhält man ein Dinitril der Formel VII, das durch selektive Hydrierung, z. B. mit einem halben Mol Natriumborhydrid, in die Verbindung II-b übergeführt werden kann.

Durch Kondensation mit β-Morpholinopropionitril kann ein Aldehyd der Formel VIII in eine Verbindung der Formel II-c übergeführt werden, die entweder direkt als Ausgangsmaterial für die erfindungsgemässe Umsetzung dienen kann, oder aber, und zwar vorzugsweise, zunächst noch durch Austausch der Morpholino- gegen die Anilinogruppe in eine Verbindung der Formel II-d übergeführt wird.

Die Umsetzung von VIII zu II-d kann auch direkt mit β-Anilinopropionitril erfolgen, doch stellt die Morpholino-Anilino-Variante den zweckmässigsten Weg zur Herstellung der Benzylpyrimidine I dar, weil hierbei unter milderen Bedingungen bessere Ausbeuten erzielt werden.

Eine Verbindung II wird dann mit Guanidin oder einem Guanidinsalz umgesetzt. Die Umsetzung kann in an sich bekannter Weise erfolgen, z. B. in einem organischen Lösungsmittel, wie einem Alkanol (Methanol, Aethanol), in Dimethylformamid, Dimethylsulfoxid oder N-Methylpyrazolon, bei einer Temperatur zwischen 25 und 200°, vorzugsweise zwischen 50 und 170°. Als Guanidinsalze können beispielsweise das Carbonat und das Hydrochlorid verwendet werden. Repräsentative Beispiele für durch das Symbol Y in der Formel II dargestellte Abgangsgruppen sind z. B. Alkoxygruppen, wie Methoxy, Aethoxy, Propoxy etc., 2-($C_{1-6}$-Alkoxy)-äthoxygruppen, wie 2-Methoxy-äthoxy und 2-Aethoxy-äthoxy, 2-Phenoxy-äthoxy, 2-Benzyloxy-äthoxy, Alkylthiogruppen, die Aminogruppe und durch aliphatische, aromatische oder heterocyclische Gruppen substituierte Aminogruppen wie Alkylamino, Benzylamino, Arylamino (z. B. gegebenenfalls substituiertes Anilino oder Naphthylamino), Dialkylamino, 1-Imidazolyl, Pyrrolidino, Piperidino, Piperazino oder Morpholino. Besonders bevorzugt als Abgangsgruppe ist der Anilinorest, dessen Phenylring gegebenenfalls ein- oder mehrfach durch Halogen, Alkyl oder Alkoxy substituiert sein kann.

Aus der DE-A-2 202 715 ist der 3,4-Dimethylthiobenzaldehyd als Zwischenprodukt bei der Herstellung von entzündungshemmend wirksamen Indenylessigsäuren bekannt.

(Siehe Schema Seite 4 ff.)

Reaktionsschema I

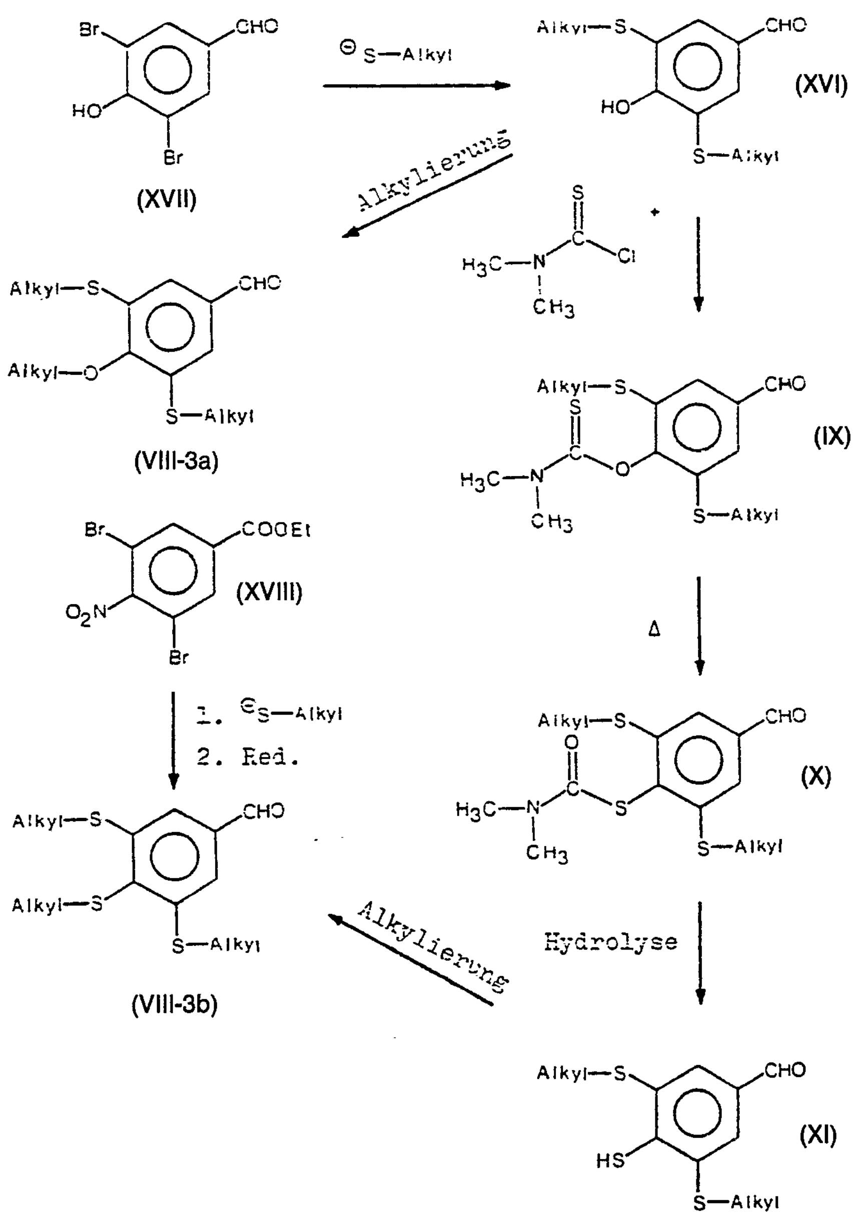


(XVII) (XVI) (VIII-3a) (IX) (XVIII) (X) (VIII-3b) (XI)

Reaktionsschema II

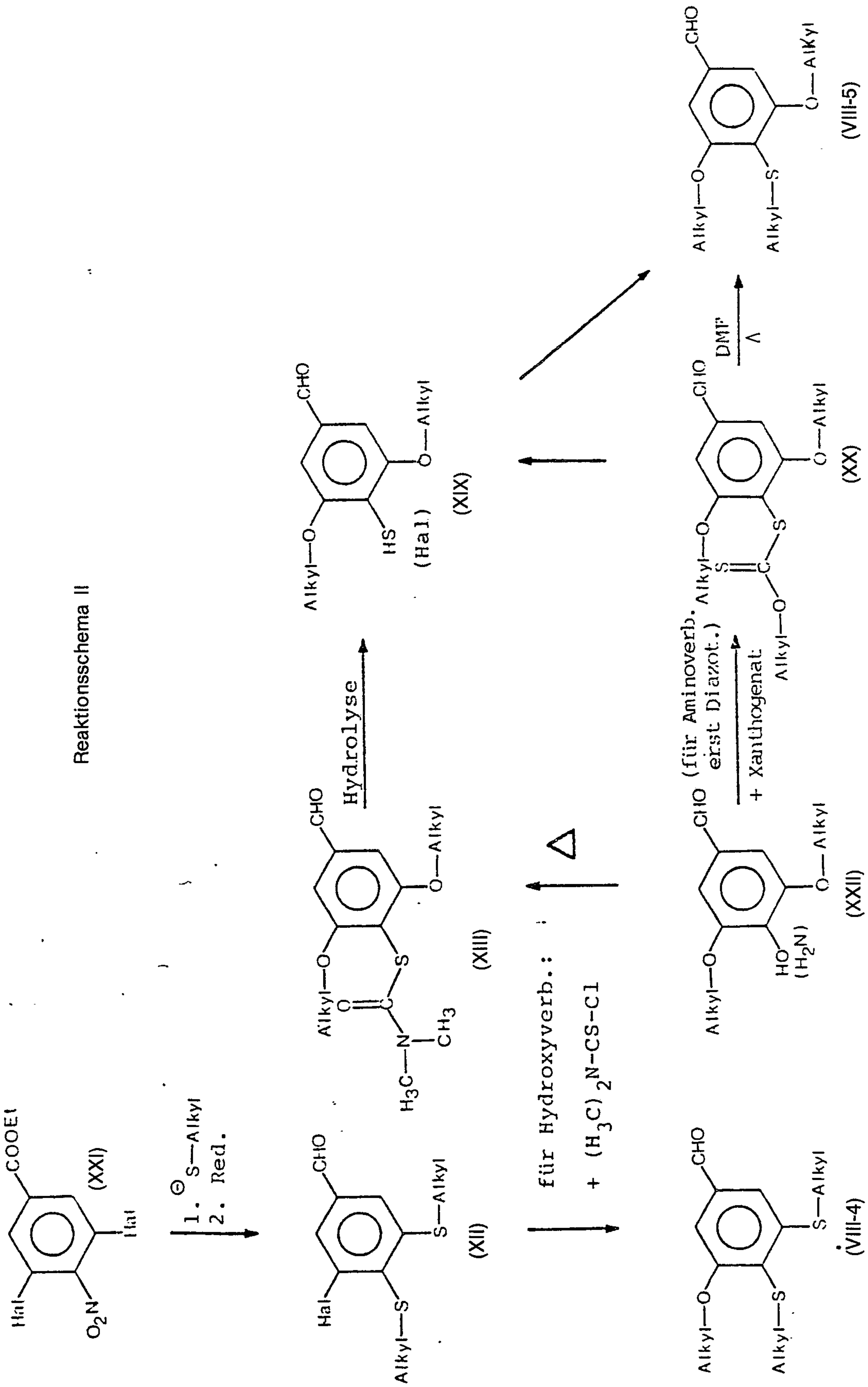


(XXI) (XII) (VIII-4) (XIII) (XXII) (XIX) (XX) (VIII-5)

Reaktionsschema III

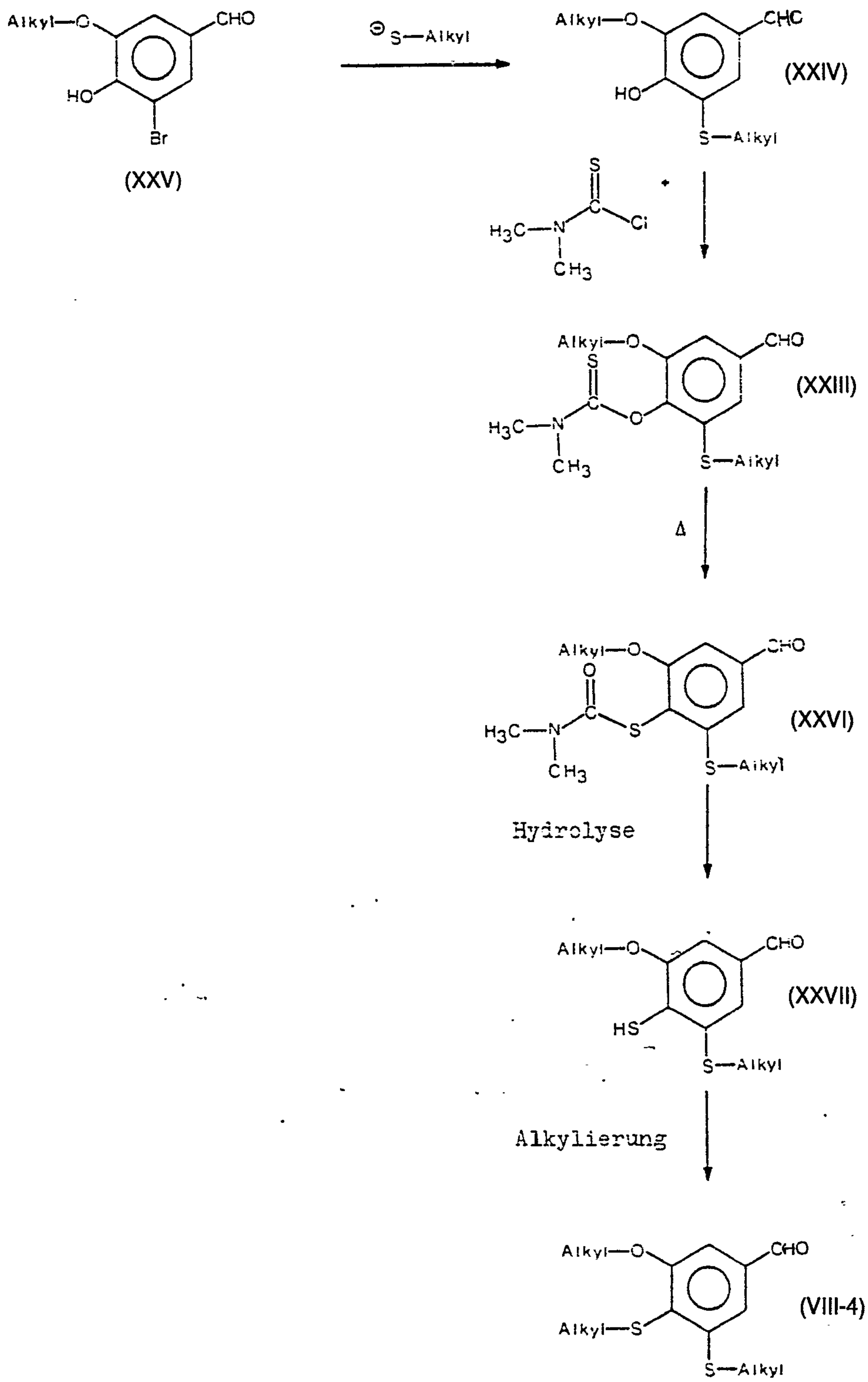

Reaktionsschema IV

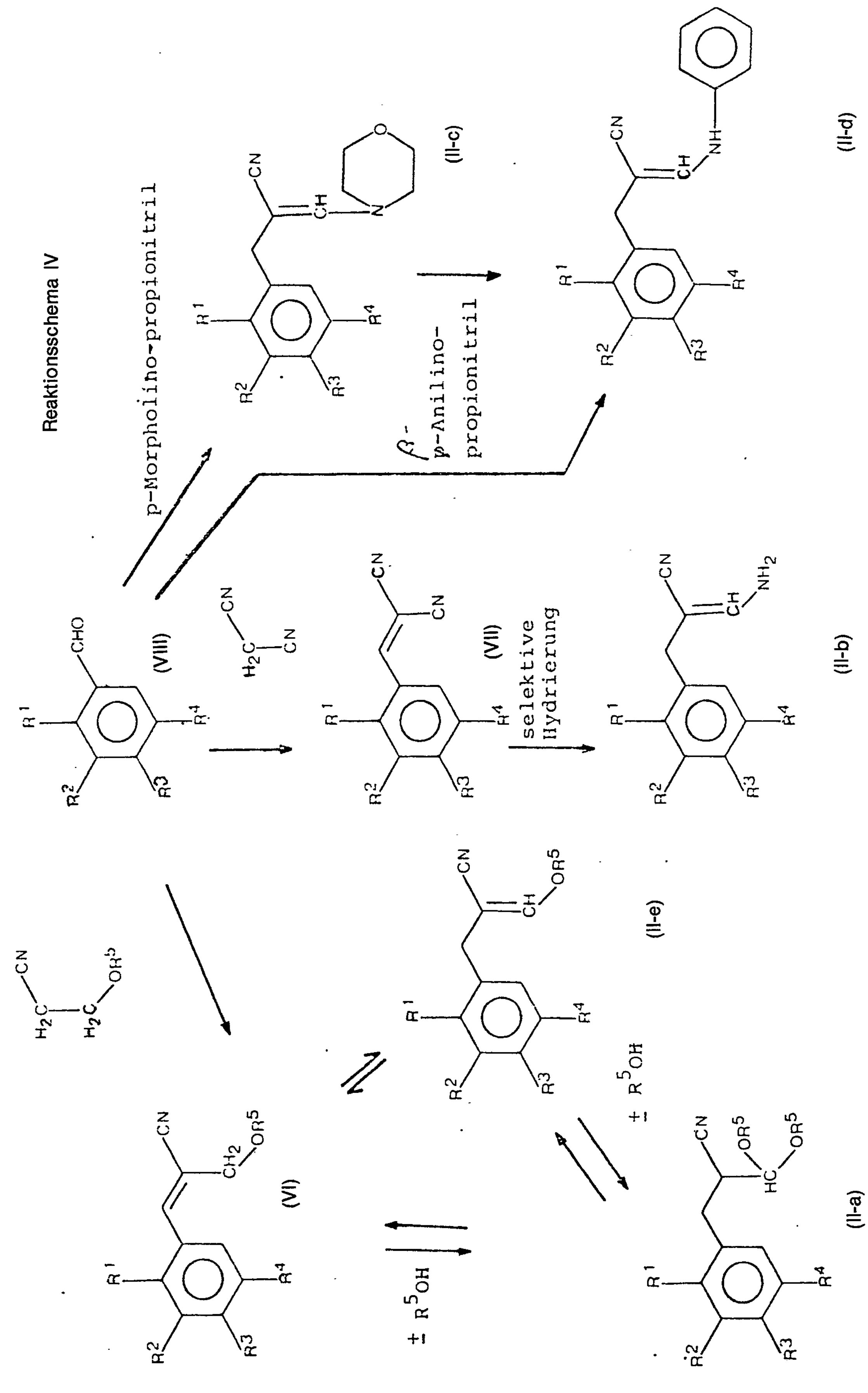


(VIII) (VII) (II-b) (II-c) (II-d) (II-e) (VI) (II-a)

Beispiel 1

Zu einer auf 0-5 °C gehaltenen Suspension von 63,3 g 4-Amino-3,5-dimethoxybenzoesäuremethylester in 300 ml Wasser und 75 ml konzentrierter Salzsäure wurde eine Lösung von 23 g Natriumnitrit in 60 ml Wasser getropft. Die nun klare orangefarbene Lösung wurde in eine eiskalte, kräftig gerührte Lösung von 80 g Kaliummethylxanthogenat in 300 ml Wasser getropft, wobei starke Gasentwicklung zu beobachten war. Nach vierstündigem Rühren bei Raumtemperatur wurde mit Essigester extrahiert, der Extrakt mit Kochsalzlösung gewaschen und über Natriumsulfat getrocknet. Aus dem nach dem Einengen verbleibenden Rückstand wurden 54 g (60 %) rohes S-[4-(Methoxycarbonyl)-2,6-dimethoxyphenyl]-O-methyldithiocarbonat, Smp. 113-115 °C, erhalten.

Ein Gemisch aus 29 g S-[4-(Methoxycarbonyl)-2,6-dimethoxyphenyl]-O-methyldithiocarbonat und 2 g Kaliummethylxanthogenat wurden in 450 ml Dimethylformamid 1 Stunde bei 100 °C gerührt. Dann wurde das Lösungsmittel unter vermindertem Druck entfernt und der Rückstand in Essigester aufgenommen. Nach Waschen mit Wasser und gesättigter Kochsalzlösung wurde über Natriumsulfat getrocknet und das Lösungsmittel abgezogen. Der Rückstand lieferte nach Umkristallisation aus Cyclohexan 18,8 g (81 %) 3,5-Dimethoxy-4-(methylthio)-benzoesäuremethylester in Form farbloser Kristalle, Smp. 86-88 °C.

Zu einer auf 0 °C gekühlten Lösung von 150 g Natriumdimethoxyäthoxyalanat (70 % in Toluol) in 150 ml Toluol wurden 50 g Morpholin in 200 ml Toluol getropft. Die klare Lösung wurde bei 0° nachgerührt und dann tropfenweise unter Rühren zu einer auf – 20 °C gekühlten Lösung von 30 g 3,5-Dimethoxy-4-(methylthio)-benzoesäuremethylester in 700 ml Toluol gegeben. Es wurde noch 5 Stunden gerührt. Anschliessend wurden langsam 100 ml Wasser zugetropft, 100 ml 3N Salzsäure zugegeben und die Phasen im Scheidetrichter getrennt. Die wässrige Phase wurde zweimal mit Essigester extrahiert, während die organischen Phasen mit Kochsalzlösung gewaschen und über Natriumsulfat getrocknet wurden. Einengen der Lösung ergab 26 g eines gelben Oeles, das durchkristallisierte und nach Umkristallisation aus Essigester/Pentan 21 g (78 %) 3,5-Dimethoxy-4-(methylthio)-benzaldehyd, Smp. 54-56 °C, lieferte.

Beispiel 2

In zu Beispiel 1 analoger Weise wurden 21,1 g 4-Amino-3,5-dimethoxybenzoesäuremethylester in 19 g (68 %) S-[4-Methoxycarbonyl)-2,6-dimethoxyphenyl]-O-äthyldithiocarbonat, Smp. 84-86 °C, übergeführt und dieses in 90 %iger Ausbeute weiter zu 3,5-Dimethoxy-4-äthylthiobenzoesäuremethylester, Smp. 50-52 °C, umgesetzt.

Zu einer bei 0 °C gerührten Lösung von 10,8 g 3,5-Dimethoxy-4-(äthylthio)-benzoesäuremethylester in 100 ml absolutem Aether wurden innert 10 Minuten 120 ml einer 20 %igen Diisobutylaluminiumhydridlösung in Hexan getropft. Es wurde während 2 Stunden bei 0-5 °C weitergerührt, nach langsamen Zutropfen von 100 ml Wasser mit 150 ml 3N Salzsäure versetzt, in Aether aufgenommen und neutralgewaschen. Die Lösung wurde über Natriumsulfat getrocknet, das Lösungsmittel abgezogen, das verbleibende gelbe Oel (11,2 g) in 100 ml Methylenchlorid aufgenommen und die Lösung in Gegenwart von 15 g Mangandioxid bei Raumtemperatur gerührt. Nach 3 Stunden wurden nochmals 15 g Mangandioxid zugegeben. Dann wurde 3 Stunden gerührt, durch ein Filtrierhilfsmittel auf der Basis von Diatomeenerde filtriert und das Filtrat eingeengt. Der Rückstand, aus Aether/Pentan kristallisiert, lieferte 9,2 g (96,5%) 3,5-Dimethoxy-4-(äthylthio)-benzaldehyd in Form farbloser Kristalle vom Smp. 38-39 °C.

Beispiel 3

Zu einer Suspension von Natriummercaptid, hergestellt aus 35,5 g 55 %iger Natriumhydrid-Dispersion in Mineralöl und 270 ml Dimethylformamid und 39,6 g Methylmercaptan in 190 ml Dimethylformamid wurden unter Stickstoff-Begasung, Rühren und Eiswasserkühlung bei 15-30 °C portionenweise 48 g 3,5-Dibrom-4-nitrobenzoesäureäthylester gegeben. Es wurde 4 Stunden bei Zimmertemperatur gerührt, in 4 l Wasser gegossen, die ausgefallenen Kristalle mit den Aetherextrakten des Filtrats vereinigt und das Lösungsmittel abgezogen. Umkristallisation des Rückstandes aus Aether/Petroläther lieferte 10,2 g 3,4,5-Tris-(methylthio)-benzoesäureäthylester. Das während der Aufarbeitung erhaltene wässrige Filtrat wurde abgekühlt und mit konzentrierter Salzsäure auf pH 2 gestellt, das feste Material abgenutscht und getrocknet. Nach Veresterung mit Aethanol/HCl wurden 28 g eines Gemisches von 3-Brom-4,5-bis-(methylthio)-benzoesäureäthylester und 3,4,5-Tris-(methylthio)-benzoesäureäthylester (Gewichtsverhältnis ca. 1 : 1) erhalten, welches durch Säulenchromatographie an Kieselgel und Methylenchlorid als Elutionsmittel aufgetrennt wurde. Man erhielt 13,8 g (32 %) 3-Brom-4,5-bis-(methylthio)-benzoesäureäthylester, Smp. 69-71 °C und 12,8 g (total also 23 g, 60 %) 3,4,5-Tris-(methylthio)-benzoesäureäthylester, Smp. 89-93 °C.

Durch Veresterung des Gemisches mit Methanol/HCl und analoge Aufarbeitung erhält man 3-Brom-4, 5-bis-(methylthio)-benzoesäuremethylester, Smp. 105-106 °C, und 3,4,5-Tris-(methylthio)-benzoesäuremethylester, Smp. 122-124 °C.

In zu Beispiel 2 analoger Weise wurden dann 35,9 g 3-Brom-4,5-bis-(methylthio)-benzoesäuremethylester in 62 %iger Ausbeute zu 3-Brom-4,5-bis-(methylthio)-benzaldehyd und 24,1 g 3,4,5-Tris-(methylthio)-benzoesäuremethylester in 58 %iger Ausbeute zu 3,4,5-Tris-(methylthio)-benzaldehyd, Smp. 160-161 °C, reduziert.

In einer anderen Ausführungsform wurden 3-Brom-4,5-bis-(methylthio)-benzoesäuremethylester und 3,4,5-Tris-(methylthio)-benzoesäuremethylester folgendermassen hergestellt :

a) Zu einer Suspension von Natriummercaptid, hergestellt aus 0,55 g 55 %iger Natriumhydrid-Dispersion (in Mineralöl) in 10 ml Dimethylformamid und 1 ml Methylmercaptan in 6 ml Dimethylformamid, wurden bei 15-25 °C, unter Stickstoffbegasung, Rühren und Eiswasserkühlung portionsweise 1,76 g 4-Nitro-3,5-dibrombenzoesäureäthylester gegeben. Das Reaktionsgemisch wurde 20 Minuten bei Zimmertemperatur gerührt, unter Hochvakuum bei 45 °C zur Trockene eingedampft, der Rückstand mit 30 ml Wasser versetzt und mit Aether extrahiert. Der Aetherextrakt wurde getrocknet, filtriert und eingedampft. Aus dem Rückstand wurden nach Umkristallisation aus heissem n-Heptan 1,5 g (94 %) 3-Brom-4,5-bis-(methylthio)-benzoesäureäthylester, Smp. 72-73 °C, erhalten.

b) Zu einer Suspension von Natriummercaptid, hergestellt aus 20,7 g 55 %iger Natriumhydrid-Dispersion (in Mineralöl) in 160 ml. Dimethylformamid und 27 ml Methylmercaptan in 110 ml Dimethylformamid, wurden bei 15-25 °C, unter Stickstoffbegasun, Rühren und Eiswasserkühlung portionsweise 28 g 4-Nitro-3,5-dibrombenzoesäureäthylester gegeben. Das Reaktionsgemisch wurde 4 Stunden bei Zimmertemperatur gerührt, unter Hochvakuum bei 45 °C zur Trockene eingedampft, der Rückstand mit 750 ml Wasser versetzt und 5 × mit je 150 ml Aether extrahiert. Der Aetherextrakt wurde getrocknet, filtriert und eingedampft. Aus dem Rückstand wurden nach Umkristallisation aus heissem n-Heptan 16,5 g (75 %) 3,4, 5-Tris-(methylthio)-benzoesäureäthylester, Smp. 90-92 °C, erhalten.

Beispiel 4

Ein Gemisch aus 22 g 4-Amino-3,5-dibrombenzoesäureäthylester, 100 ml Essigsäure und 14,4 g 2,5-Diäthoxytetrahydrofuran wurde während 1 Stunde bei 100 °C gehalten und dann unter vermindertem Druck eingeengt. Der dunkle Rückstand wurde in Essigester aufgenommen, die Lösung zunächst mit Natriumbicarbonatlösung, dann mit Kochsalzlösung gewaschen, über Natriumsulfat getrocknet und zur Trockene eingeengt. Aus Aether/Petroläther wurden 20 g (60 %) 3,5-Dibrom-4-pyrrolo-benzoesäureäthylester, Smp. 106-107 °C erhalten. 18,5 g dieser Verbindung in 40 ml warmem Dimethylformamid wurden zu einer bei Raumtemperatur gerührten Lösung von Methylmercaptid (hergestellt aus 6 g Natriumhydrid und ca. 15 g Methylmercaptan in Dimethylformamid) in 100 ml Dimethylformamid getropft. Es wurde 16 Stunden bei Raumtemperatur nachgerührt, eingeengt, in Essigester aufgenommen, mit Salzsäure auf pH 2 eingestellt, mit gesättigter Kochsalzlösung gewaschen, über Natriumsulfat getrocknet und nochmals eingeengt. Der Rückstand wurde in Aethanol/HCl verestert. Die Lösung wurde eingeengt. Aus dem Rückstand, gelöst in Aether, erhielt man nach Zugabe von Hexan 8,8 g (57 %) kristallinen 3,5-Bis-(methylthio)-4-pyrrolo-benzoesäureäthylester, Smp. 108-109 °C.

In zu Beispiel 2 analoger Weise wurden dann 21 g 3,5-Bis-(methylthio)-4-pyrrolo-benzoesäureäthylester in 67 %iger Ausbeute zu 3,5-Bis-(methylthio)-4-pyrrolo-benzaldehyd, Smp. 146-148 °C, reduziert.

Beispiel 5

Zu einer bei 0 °C gerührten Suspension von 4,7 g Natriumhydrid (50 %), das durch zweimaliges Waschen mit Pentan vom Oel befreit wurde, in 100 ml Dimethylformamid wurden tropfenweise 6,2 ml (5,0 g) Methylmercaptan zugegeben. Nach 15 Minuten wurden 12,25 g 3,5-Dimethoxy-4-brombenzaldehyd in 50 ml Dimethylformamid zugetropft. Die braune, sich aufhellende Lösung wurde bei Raumtemperatur eine halbe Stunde gerührt, auf Eiswasser gegossen und das Gemisch mit Essigester extrahiert. Der Extrakt wurde mit Wasser, kalter 1N Salzsäure und gesättigter Kochsalzlösung gewaschen, über Natriumsulfat getrocknet und das nach dem Abziehen des Lösungsmittels verbleibende gelbe Oel aus Methanol kristallisiert. Ausbeute 8,9 g (84 %) 3,5-Dimethoxy-4-(methylthio)-benzaldehyd, Smp. 55-56 °C.

**Ansprüche**

1. Aldehyde der allgemeinen Formel

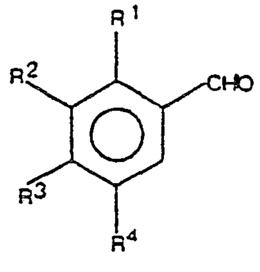

(VIII)

worin

$R^1$ Wasserstoff ; $R^2$ und $R^4$ $C_{1-6}$-Alkylthio und $R^3$ $C_{1-6}$-Alkoxy, $C_{1-6}$-Alkylthio oder Pyrrolo oder

$R^1$ Wasserstoff ; $R^2$ $C_{1-6}$-Alkoxy und $R^3$ und $R^4$ $C_{1-6}$-Alkylthio oder

$R^1$ Wasserstoff ; $R^2$ und $R^4$ $C_{1-6}$-Alkoxy und $R^3$ $C_{1-6}$-Alkylthio, Carboxy-$C_{1-6}$-alkylthio oder Cyano-$C_{1-6}$-alkylthio darstellen,

2. Aldehyde der allgemeinen Formel

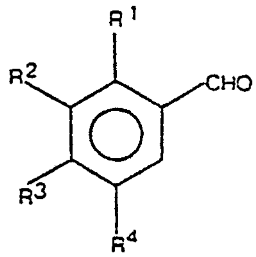

(VIII)

worin

$R^1$ Wasserstoff ; $R^2$ und $R^4$ $C_{1-6}$-Alkylthio und $R^3$ $C_{1-6}$-Alkoxy, $C_{1-6}$-Alkylthio oder Pyrrolo oder

$R^1$ Wasserstoff ; $R^2$ $C_{1-6}$-Alkoxy und $R^3$ und $R^4$ $C_{1-6}$-Alkylthio oder

$R^1$ Wasserstoff ; $R^2$ und $R^4$ $C_{1-6}$-Alkoxy und $R^3$ $C_{1-6}$-Alkylthio oder Carboxy-$C_{1-6}$-alkylthio darstellen,

3. 3,5-Dimethoxy-4-methylthiobenzaldehyd.

4. 4-Ethylthio-3,5-dimethoxybenzaldehyd.

**Claims**

1. Aldehydes of the general formula

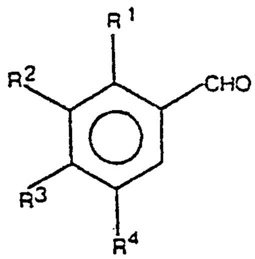

(VIII)

wherein

$R^1$ represents hydrogen ; $R^2$ and $R^4$ represent $C_{1-6}$-alkylthio and $R^3$ represents $C_{1-6}$-alkoxy, $C_{1-6}$-alkylthio or pyrrolo or

$R^1$ represents hydrogen ; $R^2$ represents $C_{1-6}$-alkoxy and $R^3$ and $R^4$ represent $C_{1-6}$-alkylthio or

$R^1$ represents hydrogen ; $R^2$ and $R^4$ represent $C_{1-6}$-alkoxy and $R^3$ represents $C_{1-6}$-alkylthio, carboxy-$C_{1-6}$-alkylthio or cyano-$C_{1-6}$-alkylthio.

2. Aldehydes of the general formula

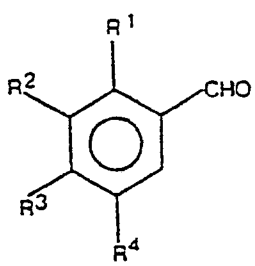

(VIII)

wherein

$R^1$ represents hydrogen ; $R^2$ and $R^4$ represent $C_{1-6}$-alkylthio and $R^3$ represents $C_{1-6}$-alkoxy, $C_{1-6}$-alkylthio or pyrrolo or

$R^1$ represents hydrogen ; $R^2$ represents $C_{1-6}$-alkoxy and $R^3$ and $R^4$ represent $C_{1-6}$-alkylthio or

$R^1$ represents hydrogen ; $R^2$ and $R^4$ represent $C_{1-6}$-alkoxy and $R^3$ represents $C_{1-6}$-alkylthio or carboxy-$C_{1-6}$-alkylthio.

3. 3,5-Dimethoxy-4-methylthiobenzaldehyde.

4. 4-Ethylthio-3,5-dimethoxybenzaldehyde.

**Revendications**

1. Aldéhydes de formule générale

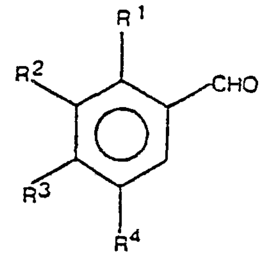

(VIII)

dans laquelle

$R^1$ représente l'hydrogène, $R^2$ et $R^4$ représentent des groupes alkylthio en C1-C6 et $R^3$ représente un groupe alcoxy en C1-C6, alkylthio en C1-C6 ou pyrrolo, ou bien

$R^1$ représente l'hydrogène, $R^2$ représente un groupe alcoxy en C1-C6 et $R^3$ et $R^4$ des groupes alkylthio en C1-C6, ou bien

$R^1$ représente l'hydrogène, $R^2$ et $R^4$ représentent des groupes alcoxy en C1-C6 et $R^3$ représente un groupe alkylthio en C1-C6, carboxy-(alkylthio en C1-C6) ou cyano-(alkylthio en C1-C6).

2. Aldéhydes de formule générale

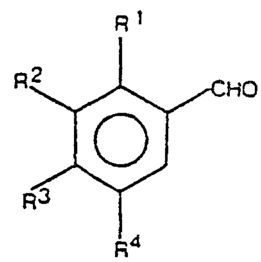

(VIII)

dans laquelle

$R^1$ représente l'hydrogène, $R^2$ et $R^4$ représentent des groupes alkylthio en C1-C6 et $R^3$ représente un groupe alcoxy en C1-C6, alkylthio en C1-C6 ou pyrrolo, ou bien

$R^1$ représente l'hydrogène, $R^2$ représente un groupe alcoxy en C1-C6 et $R^3$ et $R^4$ des groupes alkylthio en C1-C6, ou bien

$R^1$ représente l'hydrogène, $R^2$ et $R^4$ des groupes alcoxy en C1-C6 et $R^3$ un groupe alkylthio en C1-C6 ou carboxy-(alkylthio en C1-C6).

3. Le 3,5-diméthoxy-4-méthylthiobenzaldéhyde.

4. Le 4-éthylthio-3,5-diméthoxybenzaldéhyde.